# EUROPEAN PATENT SPECIFICATION

(11) **EP 3 626 300 B1**
(45) Date of publication and mention of the grant of the patent: **07.06.2023**
(21) Application number: 18820280.8
(22) Date of filing: 19.03.2018
(51) Int. Cl.: A61M 37/00, A01G 7/06, A61K 8/02, A61K 8/11, A61K 9/00, A61Q 19/00

(54) **NEEDLE FOR DISSOLVING INTO SKIN AND NEEDLE DEVICE**
NADEL ZUM AUFLÖSEN IN DER HAUT UND NADELVORRICHTUNG
AIGUILLE POUR DISSOLUTION DANS LA PEAU ET DISPOSITIF D'AIGUILLE

(30) Priority: 22.06.2017 JP 2017121980
(43) Date of publication of application: 25.03.2020
(73) Proprietor: Nangou, Norihiro, Tokyo 179-0072 (JP)
(72) Inventor: Nangou, Norihiro, Tokyo 179-0072 (JP)
(74) Representative: Pfenning, Meinig & Partner mbB
(86) International application number: PCT/JP2018/010912
(87) International publication number: WO 2018/235362

(56) References cited:
- WO-A1-2007/015441
- WO-A1-2011/042542
- WO-A1-2016/097756
- WO-A1-2016/097756
- WO-A2-2012/054582
- WO-A2-2012/103257
- JP-A- 2008 001 642
- JP-A- 2009 507 573
- JP-A- 2011 111 417
- JP-A- 2012 183 096
- JP-A- 2012 504 160
- JP-A- 2013 522 362
- JP-A- 2014 064 730
- JP-A- 2014 501 547
- JP-A- 2016 537 345
- JP-A- 2017 071 094
- JP-U- S6 398 338
- US-A- 5 395 626
- US-A1- 2009 035 446
- US-A1- 2011 177 139
- US-A1- 2011 229 529
- US-A1- 2012 220 981
- US-A1- 2012 283 695
- US-A1- 2014 243 788
- US-A1- 2017 105 928

## Description

### BACKGROUND

### 1. TECHNICAL FIELD

The present invention relates to integumental dissolving needles capable of delivering pharmaceutical or cosmetic ingredients into deep layers of integumental tissue (e.g. skin, scales, bark); and needle devices incorporating them designed to facilitate the precise administration of a desired dose, and to limit inflammation, pain, and other side effects associated with their application.

### 2. RELATED ART

Conventional intradermal dissolving microneedles ("MNs") such as those described in Patent Refs. 1 and 2 can deliver pharmaceutical or cosmetic ingredients into the upper layers of human skin (e.g. epidermis, stratum corneum), but are unable to reach the deepest layers of human skin. While large MN arrays employing a needle length of 800 µm, such as that described in Non-Patent Ref. 1, can deliver pharmaceutical or cosmetic ingredients as deep as the human dermis, they cause pain in the skin after their application. Human skin ranges from 1-4 mm in thickness (Non-Patent Ref. 2); however, cow skin is 5-7 mm in thickness, and dog skin is exceedingly thin (Non-Patent Ref. 3). This variability requires users to select MN devices having a needle length suitable for the species of interest. Moreover, conventional MN arrays do not intuitively indicate how many milligrams of ingredient(s) are present in a given unit area, nor do they employ grooves or perforations to facilitate the sectioning of the array, nor are such arrays 'pre-sectioned' for sale. The absence of such elements makes it difficult to precisely administer a desired dose. Patent Ref. 3 discloses a process of moulding arrays of microneedles, wherein particles containing pharmaceutical ingredients are embedded in tips of the microneedles. Patent Ref. 4 relates to a multilayered controlled release pharmaceutical dosage form, particularly to a dosage form adapted to water soluble drugs covered in a coating agent. Patent Ref. 8 relates to a delivery system for prophylactic, therapeutic or diagnostic agents comprised of stabilized multilamellar vesicles. Patent Refs. 5, 6, 7, 9, 10 and 11 also relate to microneedles and methods of manufacturing them, some of the disclosed microneedles being described as dissolvable.

### Related Art Documents

### Patent Literature

[Patent Reference 1] Published unexamined patent application 2010-82401. In Japanese.
[Patent Reference 2] Published unexamined patent application 2012-25723. In Japanese.
[Patent Reference 3] WO 2016/097756 A1
[Patent Reference 4] US 5 395 926 A
[Patent Reference 5] US 2009/035446 A1
[Patent Reference 6] WO 2012/054582 A2
[Patent Reference 7] US 2011/177139 A1
[Patent Reference 8] US 2011/229529 A1
[Patent Reference 9] US 2012/220981 A1
[Patent Reference 10] WO 2011/042542 A1
[Patent Reference 11] US 2012/283695 A1

### Non Patent Literature

[Non-Patent Reference 1] Advanced Science, Technology & Management Research Institute of Kyoto. [2011 Strategic Foundational Technology Improvement Support Operation, R&D Report: Development of novel tip-loaded drug-delivery microneedles, and applications to hair growth formulations.] March 2012. In Japanese. http://www.chusho.meti.go.jp/keiei/sapoin/portal/seika/2010/22h-73.pdf
[Non-Patent Reference 2] Hisashi Ishihara. [The Structure of the Skin.] 10 Apr 2015. In Japanese. http://www.ams.eng.osaka-u.ac.jp/user/ishihara/?p=432
[Non-Patent Reference 3]: Kaneko Mikihiro. [5. Learn How the Body Works, 30: Learning How the Skin Works (to Raise a Healthy Horse).] In Japanese. http://www.b-t-c.or.jp/btc_p300/btcn/btcn68/btcn068-04.pdf

### GENERAL DISCLOSURE

The present invention was developed to solve the following problems:
- How to facilitate the delivery of a pharmaceutical or cosmetic ingredient of interest into the deepest layers of integumental tissue (e.g. skin, scales, bark);
- How to limit subsequent inflammation, pain, and other side effects associated with needle device(s) ; and
- How to facilitate the precise administration of a desired amount of ingredient(s) by indicating dosage or dosages in an intuitive way, i.e. how many milligrams of the ingredient(s) are present in a given unit area.

These problems are solved by an integumental (e.g. skin, scales, bark) dissolving needle according to claim 1, which, in particular, is filled with micronized pharmaceutical ingredient(s) or micronized cosmetic ingredient(s) encapsulated by a layer of coating agent ("coating layer") that is absorbed into the integument (e.g. skin, scales, bark), to allow the said ingredient(s) to penetrate into deep layers of the integument (e.g. skin, scales, bark). Needle thickness and length may be varied according to the biological species of interest.

Also disclosed is an integumental (e.g. skin, scales, bark) dissolving needle fabricated such that suitable pharmaceutical ingredient(s) or cosmetic ingredient(s) are an integral component of the needle itself, to directly administer the said ingredient(s) without needing to wait for the needle to dissolve. Needle thickness and length may be varied according to the biological species of interest.

Also disclosed is an integumental (e.g. skin, scales, bark) dissolving needle fabricated such that suitable pharmaceutical ingredient(s) covered with a coating layer, or cosmetic ingredient(s) covered with a coating layer, are an integral component of the needle itself, to administer the said ingredient(s) (without needing to wait for the needle to dissolve) and to simplify the manufacturing process. Needle thickness and length may be varied according to the biological species of interest.

Also disclosed is an integumental (e.g. skin, scales, bark) dissolving needle device, in which needle(s) are arranged on the application-side surface of a poultice or surfaces of a poultice (e.g. hot compress, cold compress, anti-inflammatory analgesic tape), to limit subsequent inflammation, pain, and other side effects associated with needle(s).

Also disclosed is an integumental dissolving needle device, on which dosage or dosages is printed to clearly indicate how many milligrams of ingredient(s) are present in a given unit area, and which contains groove(s) or perforations in the said device to facilitate the precise administration of a desired dose. For example, an embodiment might facilitate the removal of 1 cm² unit(s), each containing 10 mg (or 1.25 mg, etc.) of a pharmaceutical ingredient, by sectioning the needle device into 1 cm² units by groove(s) or perforations, and having "10 mg" ("1.25 mg", etc.) printed on each unit. These characteristics make it easier for a user to break, cut, or otherwise divide the device and administer the desired dose.

Also disclosed is an integumental dissolving needle device, on which dosage or dosages is printed to clearly indicate how many milligrams of ingredient(s) are present in a given unit area, and which is pre-sectioned to facilitate the precise administration of a desired dose. For example, a possible embodiment is pre-sectioned, 1 cm² units, each containing 10 mg (or 1.25 mg) of a pharmaceutical ingredient, and on each of which is printed "10 mg" (or "1.25 mg", etc.). One or more units could then be applied to administer the desired dose.

The integumental (e.g. skin, scales, bark) dissolving needle, filled with micronized pharmaceutical or cosmetic ingredient(s) encapsulated by a coating agent that is absorbed into the integument (e.g. skin, scales, bark), offers the beneficial effects of allowing the encapsulated granules to penetrate deep into the integument (e.g. skin, scales, bark) once the needle itself dissolves in the integument (e.g. skin, scales, bark). This design offers superior penetrability to conventional MN(s).

The integumental dissolving needle fabricated such that suitable pharmaceutical ingredient(s) or cosmetic ingredient(s) are an integral component of the needle itself, offers the beneficial effects of allowing the said ingredient(s) to be administered directly, without waiting for the needle to dissolve, thereby allowing the said ingredient(s) to quickly penetrate into the integument.

The integumental dissolving needle fabricated such that suitable pharmaceutical ingredient(s) encapsulated by a coating layer, or suitable cosmetic ingredient(s) encapsulated by a coating layer, are an integral component of the needle itself, offers the beneficial effects of allowing the said ingredient(s) to be administered (without needing to wait for the needle to dissolve), and to simplify the manufacturing process, thereby allowing the said ingredient(s) to quickly penetrate into the integument.

The integumental (e.g. skin, scales, bark) dissolving needle device, in which any of the needles described above are arranged on the application-side surface of a poultice or surfaces of a poultice (e.g. hot compress, cold compress, anti-inflammatory analgesic tape), offers the beneficial effects of minimizing subsequent inflammation, pain, and other side effects associated with the needle(s).

The integumental dissolving needle device, on which dosage or dosages is printed and which contains groove(s) or perforations, offers the beneficial effect of facilitating the precise administration of a desired dose. For example, an embodiment might facilitate the removal of 1 cm² unit(s), each containing 10 mg (or 1.25 mg, etc.) of a pharmaceutical ingredient, by sectioning the needle device into 1 cm² units by groove(s) or perforations, and having "10 mg" ("1.25 mg", etc.) printed on each unit. These characteristics make it easier for a user to break, cut, or otherwise divide the device and administer the desired dose.

The integumental dissolving needle device, on which dosage or dosages is printed and which is pre-sectioned, offers the beneficial effect of facilitating the selection of a desired dose. For example, a possible embodiment has pre-sectioned, 1 cm² units, each containing 10 mg (or 1.25 mg) of a pharmaceutical ingredient, and each is printed with "10 mg" ("1.25 mg", etc.). One or more units could then be applied to administer the desired dose.

### BRIEF DESCRIPTION OF THE DRAWINGS

Figure 1. Integumental dissolving needle device with dosage printed on surface (groove type; perspective view)
Figure 2. Integumental dissolving needle device with dosage printed on surface (perforation type; perspective view)
Figure 3. Integumental dissolving needle device with dosage printed on surface(pre-sectioned type; perspective view)
Figure 4. Needle device housing cosmetic or pharmaceutical ingredient(s), micronized to a diameter on the micrometer order or smaller, covered with a layer of coating agent that is absorbed into the integument (cross-section view).

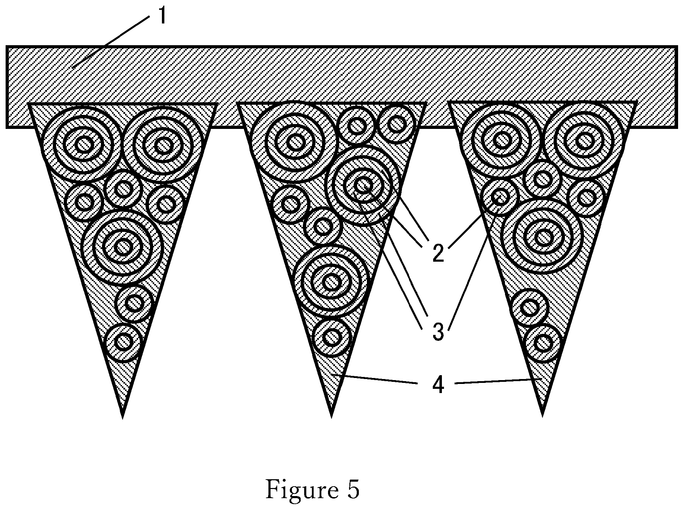

Figure 5. Needle device housing granules of different layer structures. In principle, granules may have any plural number of layers. The figure depicts a specific embodiment containing: two-layer granules, consisting of cosmetic or pharmaceutical ingredient(s), micronized to a diameter on the micrometer order or smaller, encapsulated by a layer of coating agent that is absorbed into the integument; as well as four-layer granules, consisting of the said (two-layer) granules further covered with a layer of micronized cosmetic or pharmaceutical ingredient(s), followed by another layer of coating agent (cross-section view).
Figure 6. Needle device housing multi-layer granules consisting of cosmetic or pharmaceutical ingredient(s) micronized to a diameter on the micrometer order or smaller, which are encapsulated by a layer of coating agent that is absorbed into the integument, which is further covered with a layer of micronized cosmetic or pharmaceutical ingredient(s), followed by another layer of coating agent (cross-section view).

### DESCRIPTION OF EXEMPLARY EMBODIMENTS

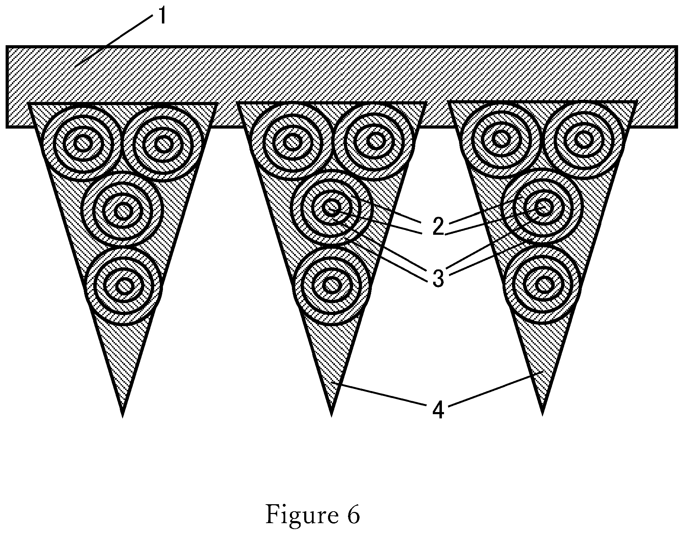

A cosmetic ingredient 2 micronized to a diameter on the micrometer order or smaller (or similarly micronized pharmaceutical ingredient 2) is encapsulated by a layer of coating agent 3 that is absorbed into the integument (e.g. skin, scales, bark). These granules are housed in an integumental (e.g. skin, scales, bark) dissolving needle 4. Multi-layer granules possess more than two layers: Figures 5 and 6 depict four-layer granules, composed of the two-layer granules described immediately above, further coated with an additional layer of micronized cosmetic (or pharmaceutical) ingredient 2, followed by an additional layer of coating agent 3. Some such capsules depicted in Figure 5, and all depicted in Figure 6, have a four-layer structure; however, granules of more than four layers are possible. Needle 4 thickness and length may be varied according to the biological species of interest.

If possible, the integumental dissolving needle 4 may be fabricated such that the pharmaceutical ingredient 2 (or cosmetic ingredient 2, or coated pharmaceutical ingredient 2, or coated cosmetic ingredient 2) is an integral component of the needle itself. In this case, the coated pharmaceutical or cosmetic ingredient 2 merely housed in the needle 4 may differ from the cosmetic ingredient 2 (or pharmaceutical ingredient 2, or coated pharmaceutical ingredient 2, or coated cosmetic ingredient 2) present in the needle's composition. For example, a needle 4 might house an encapsulated hypertension drug, while compositionally containing an antibacterial agent. Needle 4 thickness and length may be varied according to the biological species of interest.

In another embodiment, the needle(s) 4 described above may be arranged on the application-side surface of a poultice 1 or surfaces of a poultice 1 (e.g. hot compress, cold compress, anti-inflammatory analgesic tape). Alternatively, the needle(s) 4 may be arranged on a patch 1 if anti-inflammatory drug-containing poultices cannot be used (e.g. if the device is for use by a person (or species) allergic to an anti-inflammatory drug or analgesic, or a person (or species) that does not respond to the anti-inflammatory drug or analgesic).

The composition of the coating agent 3 shall include at least one of the following biocompatible substances: nucleic acid esters, nucleotides,-phosphate, polylactic acid salts, polylactic acid esters (including polylactic acid and polyglycolic acid copolymers), saccharides (including mucopolysaccharides [e.g. hyaluronic acid], dextran, maltose, glucose, sucrose, galactose, lactose, cellulose, carboxymethyl cellulose, hydroxyethyl cellulose, trehalose, peptidoglycans, polyglycolic acid, and chitin), amino acid esters, amino acid salts, proteins (e.g. gelatin, collagen, keratin), biodegradable polymers (e.g. peptides, lignin, polyvinyl alcohol, polyvinyl pyrrolidone), fullerene, vitamins, hormones, antigens, antibodies, substrates, and enzymes; alternatively or additionally, derivatives of any of these substances, and/or some mixture of them. The substances above are given as examples: any biocompatible substance capable of encapsulating the cosmetic or pharmaceutical ingredient, and being absorbed into the integument (e.g. skin, scales, bark) of the species of interest, may be used as (or in) the coating agent.

The composition of the aforementioned integumental dissolving needle 4 shall include at least one of the following biocompatible substances: nucleic acid esters, nucleotides, phosphate, polylactic acid salts, polylactic acid esters (including polylactic acid and polyglycolic acid copolymers), saccharides (including mucopolysaccharides [e.g. hyaluronic acid], dextran, maltose, glucose, sucrose, galactose, lactose, cellulose, carboxymethyl cellulose, hydroxyethyl cellulose, trehalose, peptidoglycans, polyglycolic acid, and chitin), amino acid esters, amino acid salts, proteins (e.g. gelatin, collagen, keratin), biodegradable polymers (e.g. peptides, lignin, polyvinyl alcohol, polyvinyl pyrrolidone), fullerene, vitamins, hormones, antigens, antibodies, substrates, and enzymes; alternatively or additionally, derivatives of any of these substances, and/or some mixture of them. The substances above are given as examples: any biocompatible substance capable of composing the needle, and being absorbed into the integument (e.g. skin, scales, bark) of the species of interest, may be used as (or in) the integumental dissolving needle..

Additionally, the present invention may be embodied in an integumental dissolving needle device 1, on which dosage or dosages is printed to clearly indicate how many milligrams of ingredient(s) are present in a given unit area; and which contains grooves or perforations to facilitate the separation of units, or which is pre-sectioned into the corresponding units. Groove(s) may be located on the same side of the device as the needles 4, the opposite side, or both sides. For example, such an embodiment might facilitate the removal of 1 cm² unit(s), each containing 10 mg (or 1.25 mg, etc.) of a pharmaceutical ingredient by sectioning the integumental needle device 1 into 1 cm² units by grooves or perforations, or physically pre-sectioning the device into similar unit(s), and having "10 mg" ("1.25 mg", etc.) printed on each unit. Examples

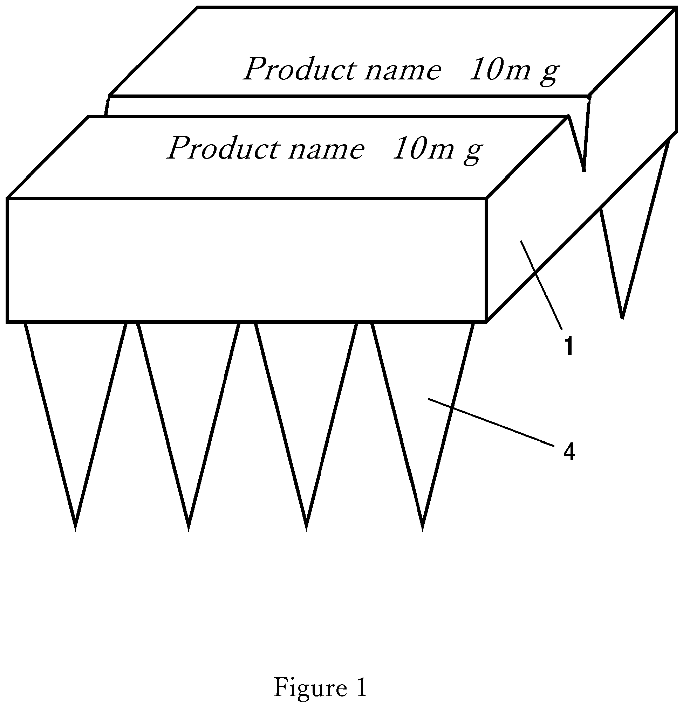

Several examples are depicted below. Possible embodiments of the present invention are not limited to those depicted in Figures 1 through 6. For example, the needle device 1 is depicted as a rectangular solid, but other shapes are possible. Figure 1 is a perspective view of a groove-type integumental (e.g. skin, scales, bark) dissolving needle device 1, with dosage or dosages printed on the surface or surfaces of each section to make explicit how many milligrams of the ingredient are present per unit area. Groove(s) may be located on the same side of the device as the needles 4, the opposite side, or both sides. As a representative example, Figure 1 depicts such a device in which each section contains 10 mg of ingredient, and in which a single groove is located on the side opposite the needles 4.

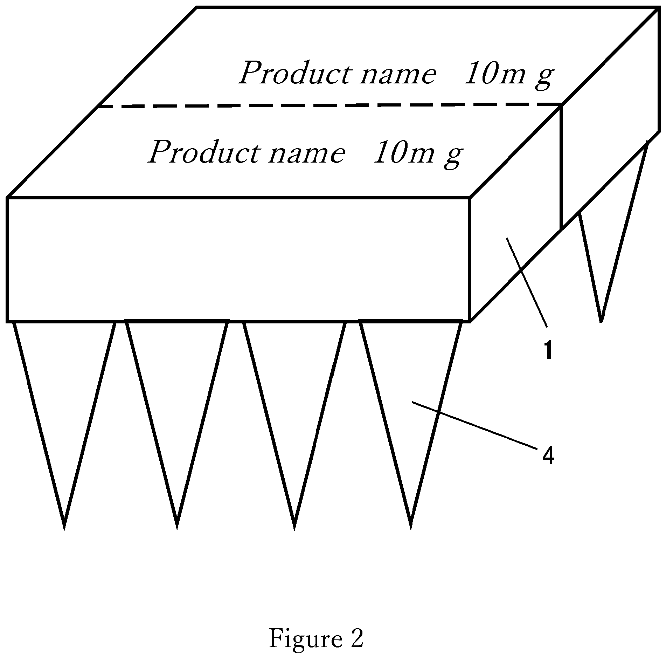

Figure 2 is a perspective view of a perforation-type integumental (e.g. skin, scales, bark) dissolving needle device 1, with dosage or dosages printed on the surface or surfaces of each section to make explicit how many milligrams of the ingredient are present per unit area. In this example, each section contains 10 mg of ingredient.

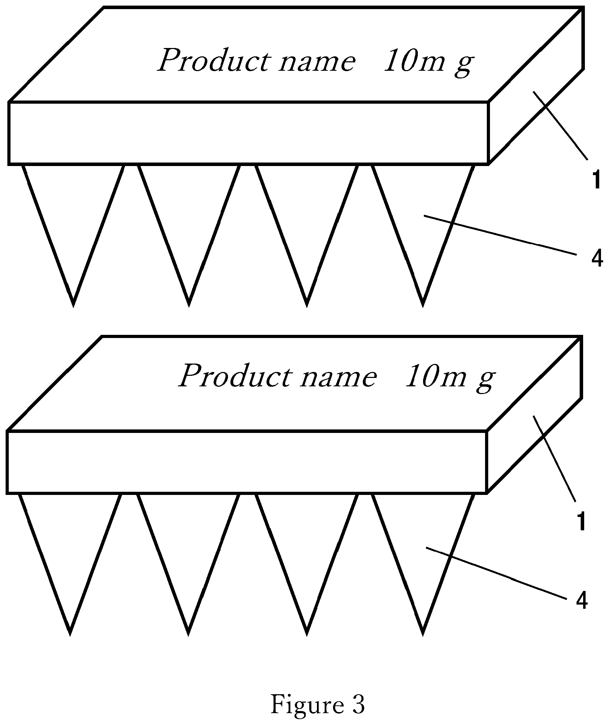

Figure 3 is a perspective view of a pre-sectioned integumental (e.g. skin, scales, bark) needle device 1, with dosage or dosages printed on the surface or surfaces of each section to make explicit how many milligrams of the ingredient are present per unit area. In this example, each section contains 10 mg of ingredient.

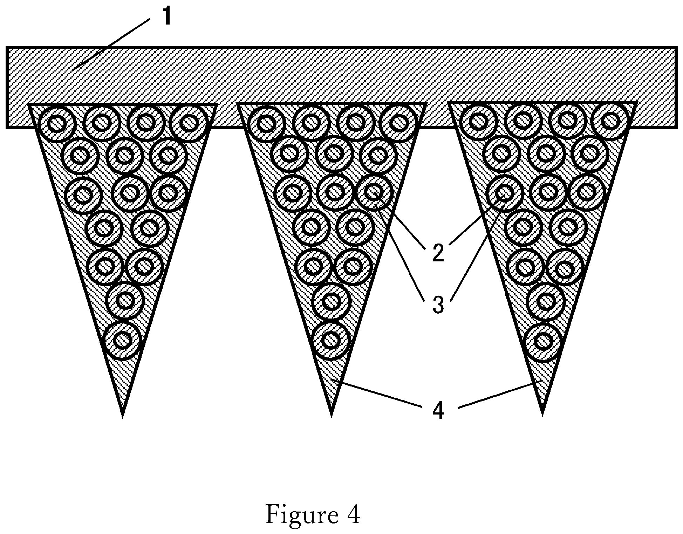

Figure 4 is a cross-section view of an integumental dissolving needle device 1, in which two-layer granules -- consisting of a cosmetic ingredient 2 micronized to a diameter on the micrometer order or smaller (or similarly micronized pharmaceutical ingredient 2), encapsulated by a layer of coating agent 3 that is absorbed into the integument (e.g. skin, scales, bark) -- are housed in the integumental (e.g. skin, scales, bark) dissolving needles 4.

Figure 5 is a cross-section view of an integumental dissolving needle device 1, in which a mixture of two-layer granules and four-layer granules -- consisting of a cosmetic ingredient 2 micronized to a diameter on the micrometer order or smaller (or similarly micronized pharmaceutical ingredient 2), encapsulated by a layer of coating agent 3 that is absorbed into the integument (e.g. skin, scales, bark), further coated with an additional layer of micronized cosmetic (or pharmaceutical) ingredient 2, followed by an additional layer of coating agent 3 -- are housed in the integumental (e.g. skin, scales, bark) dissolving needles 4. As a representative example, Figure 5 depicts some granules as having a four-layer structure; however, granules may have more than four layers.

Figure 6 is a cross-section view of an integumental dissolving needle device 1, in which four-layer granules -- consisting of a cosmetic ingredient 2 micronized to a diameter on the micrometer order or smaller (or similarly micronized pharmaceutical ingredient 2), encapsulated by a layer of coating agent 3 that is absorbed into the integument (e.g. skin, scales, bark), further coated with an additional layer of micronized cosmetic (or pharmaceutical) ingredient 2, followed by an additional layer of coating agent 3 -- are housed in the integumental (e.g. skin, scales, bark) dissolving needles 4. As a representative example, Figure 5 depicts the granules as having a four-layer structure; however, granules may have more than four layers. Industrial Applicability

The present invention is not exclusively for use for humans: it may be used for animal and plant species as well, giving it high applicability in veterinary medicine and agriculture industries.

### Reference Signs List

1. Poultice or patch
2. Micronized pharmaceutical or cosmetic ingredient
3. Coating agent
4. Needle

## Claims

1. An integumental dissolving needle (4) for delivery of one or more pharmaceutical ingredients (2), or one or more cosmetic ingredients (2), wherein
the needle houses multi-layer granules, wherein
each of the multi-layer granules comprises:
micronized pharmaceutical or cosmetic ingredients (2);
a first layer of coating agent (3) that is configured to be absorbed into an integument, the first coating layer coating the micronized pharmaceutical or cosmetic ingredients (2);
a micronized pharmaceutical or cosmetic ingredient (2) layer covering the first coating layer; and
a second coating layer containing a coating agent (3) that is configured to be absorbed into an integument, the second coating layer (3) covering the micronized pharmaceutical or cosmetic ingredient (2) layer.

2. The integumental dissolving needle (4) according to Claim 1, **characterized in that**:
the needle (4) further houses granules comprising micronized pharmaceutical or cosmetic ingredients (2), which is coated by a coating agent (3) that is configured to be absorbed into an integument.

3. The integumental dissolving needle (4) according to Claim 1, for delivery of one or more pharmaceutical ingredients (2), **characterized in that**:
the needle (4) further houses granules comprising micronized pharmaceutical ingredients (2), which is coated by a coating agent (3) that is configured to be absorbed into an integument.

4. The integumental dissolving needle (4) according to Claim 1, for delivery of one or more cosmetic ingredients (2) **characterized in that**:
the needle (4) further houses granules comprising micronized cosmetic ingredients (2), which is coated by a coating agent (3) that is configured to be absorbed into an integument.

5. The integumental dissolving needle (4) housing the granules described in one or some or all of Claims 1 to 4, **characterized in that**:
the needle (4) itself comprises one or more pharmaceutical ingredients (2), or one or more cosmetic ingredients (2), or one or more pharmaceutical ingredients (2) coated by a coating agent, or one or more cosmetic ingredients (2) coated by a coating agent (3).

6. The integumental dissolving needle (4) housing the granules described in one or some or all of Claims 1 to 4, **characterized in that**:
the needle (4) itself comprises one or more pharmaceutical ingredients (2), or one or more pharmaceutical ingredients (2) coated by a coating agent (3).

7. The integumental dissolving needle (4) housing the granules described in one or some or all of Claims 1 to 4, **characterized in that**:
the needle (4) itself comprises one or more cosmetic ingredients (2), or one or more cosmetic ingredients (2) coated by a coating agent (3).

8. The integumental dissolving needle (4) housing the granules described in one or some or all of Claims 1 to 7, wherein the integument includes a skin, a scale or a bark.

9. The integumental dissolving needle (4) of any one of Claims 1 to 8, wherein the granules further include a structure in which the micronized pharmaceutical or cosmetic ingredients (2), the coating agent (3), the another micronized pharmaceutical or cosmetic ingredient (2) layer and the coating layer (3) are repeated.

10. The integumental dissolving needle (4) of any one of Claims 1 to 9, wherein
each of the multi-layer granules consists of: the micronized pharmaceutical or cosmetic ingredients (2); the first layer of coating agent (3); the micronized pharmaceutical or cosmetic ingredient (2) layer; and the second layer of coating agent (3).

11. The integumental dissolving needle (4) of Claim 1, wherein the integumental dissolving needle (4) is for delivery of the pharmaceutical ingredient (2), wherein
each of the multi-layer granules comprises:
the micronized pharmaceutical ingredients (2);
the first layer of coating agent (3) that is configured to be absorbed into the integument, the first coating layer coating the micronized pharmaceutical ingredient (2);
the micronized pharmaceutical ingredient (2) layer covering the first coating layer; and
the second coating layer covering the micronized pharmaceutical ingredient (2) layer.

12. The integumental dissolving needle (4) of Claim 1, wherein the integumental dissolving needle (4) is for delivery of the cosmetic ingredient (2), wherein
each of the multi-layer granules comprises:
the micronized cosmetic ingredients (2);
the first layer of coating agent (3) that is configured to be absorbed into the integument, the first coating layer coating the micronized cosmetic ingredients (2);
the micronized cosmetic ingredient (2) layer covering the first coating layer; and
the second coating layer covering the micronized cosmetic ingredient (2) layer.

13. A needle device comprising one or more of the integumental dissolving needles (4) described in one or some or all of Claims 1 to 12, provided on a surface of a poultice (1) or surfaces of a poultice (1).

14. A needle device comprising one or more of the integumental dissolving needles (4) in any one of Claims 1 to 12, **characterized by**:
divisibility, and having a product name or product names and dosage or dosages written on the device surface (1) or surfaces (1).

## Patentansprüche

1. Integumentale Auflösungsnadel (4) zur Abgabe eines oder mehrerer pharmazeutischer Inhaltsstoffe (2) oder eines oder mehrerer kosmetischer Inhaltsstoffe (2), wobei
die Nadel mehrschichtige Körnchen aufnimmt, wobei jedes der mehrschichtigen Körnchen umfasst:
mikronisierte pharmazeutische oder kosmetische Inhaltsstoffe (2);
eine erste Beschichtungsmittelschicht (3), die konfiguriert ist, in ein Integument absorbiert zu werden, wobei die erste Beschichtungmittelsschicht die mikronisierten pharmazeutischen oder kosmetischen Inhaltsstoffe (2) bedeckt;
eine Schicht umfassend einen mikronisierten pharmazeutischen oder kosmetischen Inhaltsstoff (2), die die erste Beschichtungsmittelschicht bedeckt; und
eine zweite Beschichtungsmittelschicht, die ein Beschichtungsmittel (3) enthält, das konfiguriert ist, in ein Integument absorbiert zu werden, wobei die zweite Beschichtungsmittelschicht (3) die mikronisierte Schicht des pharmazeutischen oder kosmetischen Inhaltsstoffs (2) bedeckt.

2. Integumentale Auflösungsnadel (4) nach Anspruch 1, **dadurch gekennzeichnet, dass**:
die Nadel (4) ferner mikronisierte pharmazeutische oder kosmetische Inhaltsstoffe (2) umfassende Körnchen beherbergt, welche mit einem Beschichtungsmittel (3) beschichtet sind, das eingerichtet ist, in ein Integument absorbiert zu werden.

3. Integumentale Auflösungsnadel (4) nach Anspruch 1 zur Abgabe eines oder mehrerer pharmazeutischer Inhaltsstoffe (2), **dadurch gekennzeichnet, dass**:
die Nadel (4) ferner mikronisierte pharmazeutische Inhaltsstoffe (2) umfassende Körnchen beherbergt, die mit einem Beschichtungsmittel (3) beschichtet sind, das konfiguriert ist, in ein Integument absorbiert zu werden.

4. Integumentale Auflösungsnadel (4) nach Anspruch 1 zur Abgabe eines oder mehrerer kosmetischer Inhaltsstoffe (2), **dadurch gekennzeichnet, dass**:
die Nadel (4) ferner mikronisierte pharmazeutische Inhaltsstoffe (2) umfassende Körnchen aufnimmt, die mit einem Beschichtungsmittel (3) beschichtet sind, das konfiguriert ist, in ein Integument absorbiert zu werden.

5. Integumentale Auflösungsnadel (4), die die in einem oder einigen oder allen der Ansprüche 1 bis 4 beschriebenen Körnchen beherbergt, **dadurch gekennzeichnet, dass**:
die Nadel (4) selbst einen oder mehrere pharmazeutische Inhaltsstoffe (2) oder einen oder mehrere kosmetische Inhaltsstoffe (2) oder einen oder mehrere mit einem Beschichtungsmittel umhüllte pharmazeutische Inhaltsstoffe (2) oder einen oder mehrere mit einem Beschichtungsmittel (3) umhüllte kosmetische Inhaltsstoffe (2) umfasst.

6. Integumentale Auflösungsnadel (4), die die in einem oder einigen oder allen der Ansprüche 1 bis 4 beschriebenen Körnchen beherbergt, **dadurch gekennzeichnet, dass**:
die Nadel (4) selbst einen oder mehrere pharmazeutische Inhaltsstoffe (2) oder einen oder mehrere mit einem Beschichtungsmittel (3) beschichtete pharmazeutische Inhaltsstoffe (2) umfasst.

7. Integumentale Auflösungsnadel (4), die die in einem oder einigen oder allen der Ansprüche 1 bis 4 beschriebenen Körnchen beherbergt, **dadurch gekennzeichnet, dass**:
die Nadel (4) selbst einen oder mehrere kosmetische Inhaltsstoffe (2) oder einen oder mehrere mit einem Beschichtungsmittel (3) beschichtete kosmetische Inhaltsstoffe (2) umfasst.

8. Integumentale Auflösungsnadel (4), die die in einem oder einigen oder allen der Ansprüche 1 bis 7 beschriebenen Körnchen aufnimmt, wobei das Integument eine Haut, eine Schuppe oder Rinde umfasst.

9. Integumentale Auflösungsnadel (4) nach einem der Ansprüche 1 bis 8, wobei die Körnchen ferner eine Struktur umfassen, in der die mikronisierten pharmazeutischen oder kosmetischen Inhaltsstoffe (2), das Beschichtungsmittel (3), die andere einen mikronisierten pharmazeutischen oder kosmetischen Inhaltsstoff (2) umfassende Schicht und die Beschichtungsschicht (3) sich wiederholen.

10. Integumentale Auflösungsnadel (4) nach einem der Ansprüche 1 bis 9, wobei
jedes der mehrschichtigen Körnchen besteht aus: den mikronisierten pharmazeutischen oder kosmetischen Inhaltsstoffen (2); der ersten Beschichtungsmittelschicht (3); der Schicht aus dem mikronisierten pharmazeutischen oder kosmetischen Inhaltsstoff (2); und der zweiten Beschichtungsmittelschicht (3).

11. Integumentale Auflösungsnadel (4) nach Anspruch 1, wobei die integumentale Auflösungsnadel (4) zur Abgabe des pharmazeutischen Inhaltsstoffs (2) vorgesehen ist, wobei
jedes der mehrschichtigen Körnchen umfasst:
die mikronisierten pharmazeutischen Inhaltsstoffe (2);
die erste Beschichtungsmittelschicht (3), die konfiguriert ist, in das Integument absorbiert zu werden, wobei die erste Beschichtungsschicht den mikronisierten pharmazeutischen Inhaltsstoff (2) beschichtet;
die einen mikronisierten pharmazeutischen Inhaltsstoff (2) umfassende Schicht, die die erste Beschichtungsschicht bedeckt; und
die zweite Beschichtungsschicht, die die den mikronisierten pharmazeutischen Inhaltsstoff (2) umfassende Schicht bedeckt.

12. Integumentale Auflösungsnadel (4) nach Anspruch 1, wobei die Integumentale Auflösungsnadel (4) zur Abgabe des kosmetischen Inhaltsstoffs (2) vorgesehen ist, wobei
jedes der mehrschichtigen Körnchen umfasst:
die mikronisierten kosmetischen Inhaltsstoffe (2);
die erste Beschichtungsmittelschicht (3), die konfiguriert ist, in das Integument absorbiert zu werden, wobei die erste Beschichtungsschicht die mikronisierten kosmetischen Inhaltsstoffe (2) beschichtet;
die einen mikronisierten kosmetischen Inhaltsstoff (2) umfassende Schicht, die die erste Beschichtungsschicht bedeckt; und
die zweite Beschichtungsschicht, die die Schicht aus mikronisiertem kosmetischem Inhaltsstoff (2) bedeckt.

13. Nadelvorrichtung, die eine oder mehrere der in einem oder einigen oder allen der Ansprüche 1 bis 12 beschriebenen integumentalen Auflösungsnadeln (4) umfasst, die auf einer Oberfläche einer Heilpackung (1) oder auf Oberflächen einer Heilpackung (1) vorgesehen ist.

14. Nadelvorrichtung, die eine oder mehrere der integumentalen Auflösungsnadeln (4) nach einem der Ansprüche 1 bis 12 umfasst, **gekennzeichnet durch**:
Teilbarkeit und **dadurch, dass** sie einen Produktnamen oder Produktnamen und eine Dosierung oder Dosierungen aufweist, die auf die Vorrichtungsoberfläche (1) oder -oberflächen (1) geschrieben sind.

## Revendications

1. Aiguille de dissolution tégumentale (4) pour l'administration d'un ou plusieurs ingrédients pharmaceutiques (2), ou d'un ou plusieurs ingrédients cosmétiques (2), dans laquelle
l'aiguille loge des granules multicouches, dans laquelle
chacun des granules multicouches comprend :
ingrédients pharmaceutiques ou cosmétiques micronisés (2) ;
une première couche d'agent de revêtement (3) qui est configurée pour être absorbée dans un tégument, la première couche de revêtement recouvrant les ingrédients pharmaceutiques ou cosmétiques micronisés (2) ;
une couche d'ingrédient pharmaceutique ou cosmétique micronisé (2) recouvrant la première couche de revêtement ; et
et une seconde couche de revêtement contenant un agent de revêtement (3) qui est configuré pour être absorbé dans un tégument, la seconde couche de revêtement (3) recouvrant la couche d'ingrédient pharmaceutique ou cosmétique micronisé (2).

2. Aiguille de dissolution tégumentale (4) selon la revendication 1, **caractérisée en ce que** :
l'aiguille (4) loge en outre des granulés comprenant des ingrédients pharmaceutiques ou cosmétiques micronisés (2), qui sont recouverts par un agent de revêtement (3) qui est configuré pour être absorbé dans un tégument.

3. Aiguille à dissolution tégumentale (4) selon la revendication 1, pour la délivrance d'un ou plusieurs ingrédients pharmaceutiques (2), **caractérisée en ce que** :
l'aiguille (4) loge en outre des granulés comprenant des ingrédients pharmaceutiques micronisés (2), qui sont recouverts par un agent de revêtement (3) qui est configuré pour être absorbé dans un tégument.

4. Aiguille de dissolution tégumentale (4) selon la revendication 1, pour la délivrance d'un ou plusieurs ingrédients cosmétiques (2) **caractérisée en ce que** :
l'aiguille (4) loge en outre des granulés comprenant des ingrédients cosmétiques micronisés (2), qui sont recouverts par un agent de revêtement (3) qui est configuré pour être absorbé dans un tégument.

5. Aiguille de dissolution tégumentale (4) logeant les granulés décrite dans l'une des ou certaines des ou toutes les revendications 1 à 4, **caractérisée en ce que** :
l'aiguille (4) elle-même comprend un ou plusieurs ingrédients pharmaceutiques (2), ou un ou plusieurs ingrédients cosmétiques (2), ou un ou plusieurs ingrédients pharmaceutiques (2) recouvert(s) par un agent de revêtement, ou un ou plusieurs ingrédients cosmétiques (2) recouvert(s) d'un agent de revêtement (3).

6. Aiguille de dissolution tégumentale (4) logeant les granulés décrite dans l'une des ou certaines des ou toutes les revendications 1 à 4, **caractérisée en ce que** :
l'aiguille (4) elle-même comprend un ou plusieurs ingrédients pharmaceutiques (2), ou un ou plusieurs ingrédients pharmaceutiques (2) recouvert(s) par un agent de revêtement (3).

7. Aiguille de dissolution tégumentale (4) logeant les granulés décrite dans l'une des ou certaines des ou toutes les revendications 1 à 4, **caractérisée en ce que** :
l'aiguille (4) comprend elle-même un ou plusieurs ingrédients cosmétiques (2), ou un ou plusieurs ingrédients cosmétiques (2) recouvert(s) par un agent de revêtement (3).

8. Aiguille de dissolution tégumentaire (4) logeant les granulés décrits dans une des ou plusieurs des ou toutes les revendications 1 à 7, dans laquelle le tégument comprend une peau, une écaille ou une écorce.

9. Aiguille de dissolution tégumentale (4) selon l'une quelconque des revendications 1 à 8, dans laquelle les granulés comprennent en outre une structure dans laquelle les ingrédients pharmaceutiques ou cosmétiques micronisés (2), l'agent de revêtement (3), l'autre ingrédient pharmaceutique ou cosmétique micronisé (2) et la couche de revêtement (3) sont répétées.

10. Aiguille de dissolution tégumentale (4) selon l'une quelconque des revendications 1 à 9, dans laquelle chacun des granulés multicouches consiste en : les ingrédients pharmaceutiques ou cosmétiques micronisés (2) ; la première couche d'agent de revêtement (3) ; la couche micronisée d'ingrédient pharmaceutique ou cosmétique (2) ; et la seconde couche d'agent de revêtement (3).

11. Aiguille de dissolution tégumentale (4) selon la revendication 1, dans laquelle l'aiguille de dissolution tégumentale (4) est destinée à l'administration de l'ingrédient pharmaceutique (2), dans laquelle
chacun des granulés multicouches comprend :
les ingrédients pharmaceutiques micronisés (2) ;
la première couche d'agent de revêtement (3) qui est configurée pour être absorbée dans le tégument, la première couche de revêtement recouvrant l'ingrédient pharmaceutique micronisé (2) ;
la couche d'ingrédient pharmaceutique micronisé (2) recouvrant la première couche de revêtement ; et
la seconde couche de revêtement recouvrant la couche d'ingrédient pharmaceutique micronisé (2).

12. Aiguille de dissolution tégumentale (4) selon la revendication 1, dans laquelle l'aiguille de dissolution tégumentale (4) est destinée à l'administration de l'ingrédient cosmétique (2), dans laquelle
chacun des granulés multicouches comprend :
les ingrédients cosmétiques micronisés (2) ;
la première couche d'agent de revêtement(3) qui est configurée pour être absorbée dans le tégument, la première couche de revêtement recouvrant les ingrédients cosmétiques micronisés (2) ;
la couche d'ingrédient cosmétique micronisé (2) recouvrant la première couche de revêtement ; et
la seconde couche de revêtement recouvrant la couche d'ingrédient cosmétique micronisé (2).

13. Dispositif à aiguille comprenant une ou plusieurs des aiguilles à dissolution tégumentale (4) décrites dans l'une ou certaines des ou toutes les revendications 1 à 12, prévues sur une surface d'un cataplasme (1) ou des surfaces d'un cataplasme (1).

14. Dispositif à aiguille comprenant une ou plusieurs des aiguilles à dissolution tégumentale (4) selon l'une quelconque des revendications 1 à 12, **caractérisé par** :
divisibilité, et ayant un nom de produit ou des noms de produit et un dosage ou des dosages écrits sur la surface (1) ou les surfaces (1) du dispositif.
